**Europäisches Patentamt**

**European Patent Office**

**Office européen des brevets**

(19)

(11) Veröffentlichungsnummer: **0 044 804**

**A1**

## EUROPÄISCHE PATENTANMELDUNG

(12)

(21) Anmeldenummer: **81730068.4**

(22) Anmeldetag: **18.07.81**

(51) Int. Cl.³: **A 61 M 25/00**

(30) Priorität: **21.07.80 DE 3027569**

(43) Veröffentlichungstag der Anmeldung:
**27.01.82 Patentblatt 82/4**

(84) Benannte Vertragsstaaten:
**AT BE CH DE FR GB IT LI LU NL SE**

(71) Anmelder: **Barsom, Shafik, Dr. med.**
**Geibelstrasse 54**
**D-3000 Hannover(DE)**

(72) Erfinder: **Barsom, Shafik, Dr. med.**
**Geibelstrasse 54**
**D-3000 Hannover(DE)**

(74) Vertreter: **Eikenberg & Brümmerstedt Patentanwälte**
**Schackstrasse 1**
**D-3000 Hannover(DE)**

(54) **Blasen-Dauerkatheter.**

(57) Es handelt sich um ein Blasen-Dauerkatheter mit einem in die Blase mündenden Hauptkanal, der mit einem Verschluß versehen ist, und ggfs. mit einer nahe dem inneren Ende angeordneten aufblasbaren Kammer, deren Expansionszustand über einen Verbindungskanal von außen regelbar ist. Erfindungsgemäß ist im Katheter noch ein zusätzlicher, von außen zugänglicher Zufuhrkanal gebildet, der im eingesetzten Zustand des Katheters noch vor der Prostata in die Harnröhre mündet und zur Zufuhr eines medizinischen Behandlungsmittels in die Harnröhre dient. Dieser Zufuhrkanal besitzt im einfachsten Fall eine einzige Auslaßöffnung, aber zweckmäßig ist vorgesehen, daß der Zufuhrkanal im Bereich seiner harnröhrenseitigen Mündung in einen Ringkanal mündet, der mehrere über den Katheterumfang verteilte Auslässe besitzt. Zusätzlich oder alternativ kann auch vorgesehen sein, daß der Zufuhrkanal schraubenlinienförmig um den Katheterumfang herumgeführt und mit mehreren im Abstand voneinander angeordneten Auslässen versehen ist.

FIG.1

EP 0 044 804 A1

Shafik Barsom, Dr. med                    441/6 EU

Blasen-Dauerkatheter

Bei krankhafter Vergrößerung der Prostata kommt es
zu einer zunehmenden Verengung der blasenseitigen Mündung
der Harnröhre, die bis zum völligen Verschluß dieser Harnröhrenmündung führen kann. Die Folge ist, daß die Entleerung
der Blase auf natürlichem Wege zunehmend erschwert oder sogar ganz unmöglich wird. In solchen Fällen muß ein Blasen-
Dauerkatheter in die Harnröhre des betreffenden Patienten
eingesetzt werden.

Es sind bereits viele verschiedene Typen von Blasen-Dauerkathetern bekannt, denen gemeinsam ist, daß sie aus
einer Röhre bestehen, welche an der Prostata vorbei bis in
die Blase eingeschoben wird und an ihrem äußeren, aus der
Harnröhre herausragenden Ende mit einem Verschluß versehen
ist. Eine besonders häufig verwendete Type dieser Katheter
ist aus einem weichen, gummiartigen Material hergestellt und
besitzt nahe seinem inneren Ende noch eine im allgemeinen
ringförmige Kammer, die über einem gesonderten Kanal nach
außen in Verbindung steht. Im Gebrauch wird diese Kammer bis
in die Blase eingeschoben und dann von außen über ihren Verbindungskanal ballonartig ausgedehnt, wodurch sich eine Sicherung des Katheters gegen ein unbeabsichtigtes Herausrutschen ergibt.

- 2 -

Diese Katheter haben sich von ihrer Funktion her
durchaus bewährt. Sie stellen jedoch einen Fremdkörper im
Körper des Patienten dar, und daraus resultieren eine Reihe
von Nachteilen. Insbesondere können sich Strikturen der Harnröhre und/oder eine Infektion und Entzündung der Harnröhrenwand einstellen, welche zur fiebrigen Erkrankung des Patienten ("Katheterfieber") und zur Absonderung eines eiterigen
Sekrets nach außen führen sowie auch Anlaß zu einer "steigenden Infektion" (von der Harnröhre über die Blase bis zu
den Nieren) geben kann. Begünstigt wird die Infektion und
Entzündung der Harnröhrenwand dabei durch die Tatsache, daß
beim Wechseln des Katheters, das normalerweise alle 14 Tage
erfolgt, ein sog. "Sandpapierphänomen" auftritt, worunter
zu verstehen ist, daß Harnkristalle, die sich am inneren, in
der Blase befindlichen Ende des Katheters ablagern, in die
Harnröhre gelangen, wo sie zwischen dem Katheter und der
Harnröhrenwand eine starke Scheuerwirkung ausüben.

Mit der Erfindung sollen diese Nachteile der bekannten Katheter vermieden oder zumindest stark abgeschwächt werden. Das wird erfindungsgemäß dadurch erreicht, daß im Katheter ein zusätzlicher, von außen zugänglicher Zufuhrkanal
gebildet ist, der beim eingesetzten Zustand des Katheters noch
vor der Prostata in die Harnröhre mündet und zur Zufuhr eines
medizinischen Behandlungsmittels in die Harnröhre dient.

Es hat sich gezeigt, daß mit Hilfe dieses zusätzlichen Zufuhrkanals im Katheter tatsächlich den vorangehend geschilderten Nachteilen entgegengewirkt werden kann, denn es
gelingt mit Hilfe dieses zusätzlichen Zufuhrkanals erstmals,
bei Bedarf oder in regelmäßigen Zeitabständen die Harnröhre
unmittelbar mit einem medizinischen Behandlungsmittel zu behandeln. Dieses Behandlungsmittel kann dabei verschiedenartig beschaffen sein. Im einfachsten Fall ist es ein Spülmittel zum Lösen und zum Ausspülen der gebildeten Harnkristalle. Zweckmäßig ist auch ein antibiotisch wirkendes Mittel, welches zugleich auch noch schmierende Eigenschaften hat. Durch das Einführen dieses Mittels in die Harnröhre, das je nach Lage des Falles vom behandelnden Arzt oder vom Patienten selbst vorgenommen werden kann, wird dem Entstehen von Infektionen und Entzündungen vorgebeugt (bzw. bereits entstandene Infektionen und Entzündungen werden beseitigt), und dadurch verschwinden auch deren Folgeerscheinungen. Weiterhin sorgt die Schmiereigenschaft dieses Mittels auch noch dafür, daß das Katheter innerhalb der Harnröhre ständig von einem weichen Film umgeben ist, der das Sandpapierphänomen beim Wechseln des Katheters vermindert. Außerdem wird dem Auftreten von Strikturen eingegengewirkt, weil einerseits die Druckreibung des Katheters auf die Harnröhrenwand beträchtlich herabgesetzt wird und andererseits auch keine entzündungsbedingten Vernarbungen mehr auftreten.

Im Prinzip genügt es, den Zufuhrkanal mit nur einer einzigen Auslaßöffnung in die Harnröhre münden zu lassen, aber bevorzugt ist der Zufuhrkanal mit mehreren Auslaßöffnungen versehen. Dies kann auf unterschiedliche Weise bewirkt werden, beispielsweise dadurch, daß der Zufuhrkanal im Bereich seiner harnröhrenseitigen Mündung in einen Ringkanal mündet, der mehrere über den Katheterumfang verteilte Auslässe besitzt. Zusätzlich oder auch alternativ kann auch vorgesehen sein, daß der Zufuhrkanal schraubenlinienförmig um den Katheterumfang herumgeführt und mit mehreren im Abstand voneinander angeordneten Auslässen versehen ist.

- 4 -

Nachfolgend wird die Erfindung in Ausführungsbeispielen anhand der Zeichnungen näher erläutert. Dabei stellen dar:

Fig. 1 schematisch eine Harnröhre eines Mannes mit eingesetztem Katheter,

Fig. 2 das Katheter gemäß Fig. 1 im Querschnitt (Ebene II-II in Fig. 3),

Fig. 3 das Mittelteil des Katheters gemäß Fig. 1 im Längsschnitt (Ebene III-III in Fig. 2),

Fig. 4 das Mittelteil einer gegenüber Fig. 1 abgewandelten Ausführungsform des Katheters.

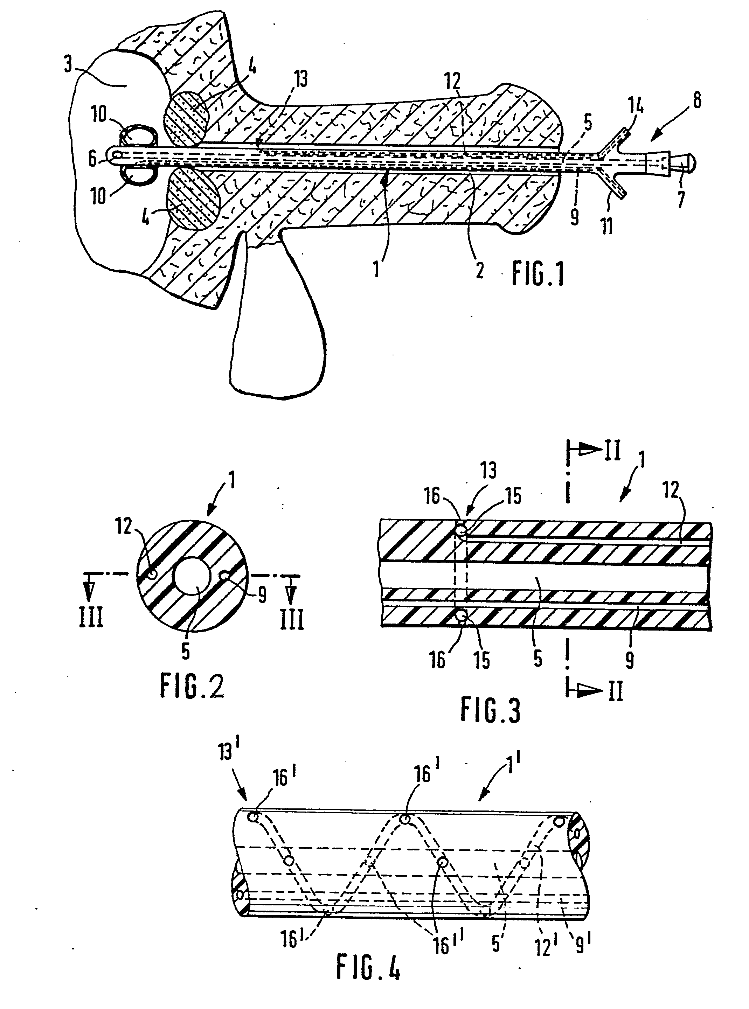

Die Fig. 1 läßt schematisch die Lage eines Blasen-Dauerkatheters 1 bei einem an Prostatahypertrophie leidenden Mann erkennen. Das Katheter 1 ist von außen in die Harnröhre 2 eingeführt, und zwar an der Prostata 4 vorbei bis in die Blase 3 hinein. Es besitzt einen Hauptkanal 5, der innerhalb der Blase eine Öffnung 6 aufweist und am äußeren Ende 8 des Katheters durch einen Verschlußstopfen 7 verschließbar ist. Nahe der inneren Öffnung 6 befindet sich eine ballonartig aufblasbare Kammer 10, die über einen gesonderten Verbindungskanal 9 mit z. B. Wasser gefüllt worden ist. Der Verbindungskanal 9 mündet am äußeren Katheterende 8 in einem gesonderten Kopfteil 11, das mit einem ventilartigen Verschluß versehen ist. Das soweit beschriebene Katheter ist bekannt und üblich, so daß auf seine Handhabung und Funktionsweise nicht mehr näher eingegangen zu werden braucht.

Neu ist an dem Katheter 1, daß es noch einen weiteren Kanal 12 zur Zufuhr eines Behandlungsmittels in die Harnröhre 2 aufweist, der in Fig. 2 und 3 näher dargestellt ist. Als Einlaß für diesen Zufuhrkanal 12 ist am äußeren Katheterende 8 noch ein zweites Kopfteil 14 vorgesehen, das analog
dem Kopfteil 11 mit einem ventilartigen Verschluß versehen
sein kann. Der Zufuhrkanal 12 ist ähnlich dem Verbindungskanal 9 ausgebildet, er öffnet sich jedoch innerhalb der
Harnröhre 2, also noch vor der Prostata 4, nach außen. Die
Mündung 13 des Zufuhrkanals 12 ist dabei im einfachsten Fall
eine einzige Auslaßöffnung, indem der Kanal 12 lediglich
durch die äußere Katheterwand hindurchgeführt ist. Wie in
Fig. 3 gezeigt, ist aber zweckmäßig im Bereich der Mündung 13
noch ein Ringkanal 15 vorgesehen, der mit dem Kanal 12 in
Verbindung steht und mehrere über den Katheterumfang verteilte kleine Auslässe 16 besitzt.

Eine abgewandelte Ausführungsform des Katheters ist
in Fig. 4 dargestellt, wobei gleiche Teile mit gleichen, aber
gestrichenen Bezugszeichen bezeichnet sind. In dieser Ausführungsform gemäß Fig. 4 ist der Zufuhrkanal 12' schraubenlinienförmig um den Katheterumfang herumgeführt und mit mehreren
im Abstand voneinander angeordneten Auslässen 16' versehen.

Im Gebrauch des Katheters 1 wird entweder bei Bedarf
oder aber in regelmäßigen Zeitabständen das Behandlungsmittel
durch das Kopfteil 14 und den Zufuhrkanal 12 in die Harnröhre
2 injiziert. Dort verteilt es sich, was durch Drehen bzw. Hin-
und Herschieben des Katheters während des Injizierens des
Behandlungsmittels noch unterstützt werden kann. Die Menge des
Behandlungsmittels kann dabei so bemessen sein, daß der Zwischenraum zwischen der Harnröhrenwand und dem Katheter völlig
ausgefüllt wird, aber es kann in vielen Fällen auch eine geringere Menge an Behandlungsmittel ausreichend sein bzw. in
anderen Fällen ein Spülmittel im Überschuß appliziert werden.

KRE/s                                              ----

Patentansprüche
--------------------------------

1.      Blasen-Dauerkatheter mit einem in die Blase mündenden Hauptkanal, der außen mit einem Verschluß versehen ist,
und ggfs. mit einer nahe dem inneren Ende angeordneten aufblasbaren Kammer, deren Expansionszustand über einen Verbindungskanal von außen regelbar ist, dadurch gekennzeichnet,
daß im Katheter (1) ein zusätzlicher, von außen zugänglicher
Zufuhrkanal (12) gebildet ist, der im eingesetzten Zustand
des Katheters noch vor der Prostata in die Harnröhre (2)
mündet und zur Zufuhr eines medizinischen Behandlungsmittels
in die Harnröhre dient.

2.      Blasen-Dauerkatheter nach Anspruch 1, dadurch gekennzeichnet, daß der Zufuhrkanal (12) im Bereich seiner harnröhrenseitigen Mündung (13) in einen Ringkanal (15) mündet,
der mehrere über den Katheterumfang verteilte Auslässe (16)
besitzt.

3.      Blasen-Dauerkatheter nach Anspruch 1 oder 2, dadurch
gekennzeichnet, daß der Zufuhrkanal (12') schraubenlinienförmig um den Katheterumfang herumgeführt und mit mehreren im
Abstand voneinander angeordneten Auslässen (16') versehen ist.

0044804

1/1

FIG.1

FIG.2

FIG.3

FIG.4

0044804

Nummer der Anmeldung
EP 81 73 0068

Europäisches
Patentamt

**EUROPÄISCHER RECHERCHENBERICHT**

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 61 M 25/00 |
| X | <u>DE - A - 2 737 855</u> (GINDELE)<br>* Seite 5, Absatz 2; Figuren *<br><br>-- | 1 | |
| X | <u>DE - A - 2 632 280</u> (HOECHST)<br>* Seite 5, Zeilen 14-22; Figur *<br><br>-- | 1,2 | |
| | <u>US - A - 3 593 713</u> (BOGOFF)<br>* Spalte 1, Zeilen 40-61; Fi-<br>guren *<br><br>---- | 1,2 | RECHERCHIERTE SACHGEBIETE (Int. Cl.³)<br><br>A 61 M |

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 19-10-1981 | VANRUNXT |

EPA form 1503.1  06.78